# EUROPÄISCHE PATENTSCHRIFT

(11) **EP 2 809 644 B1**
(45) Veröffentlichungstag und Bekanntmachung des Hinweises auf die Patenterteilung: **28.09.2016**
(21) Anmeldenummer: 13701282.9
(22) Anmeldetag: 28.01.2013
(51) Int. Cl.: C07C 201/08, C07C 205/06, C01B 17/94, C01B 17/88

(54) **VERFAHREN UND ANLAGE ZUR HERSTELLUNG VON NITROBENZOL**
METHOD AND DEVICE FOR MANUFACTURING NITROBENZOL
PROCÉDÉ ET INSTALLATION DESTINÉS À LA FABRICATION DE NITROBENZÈNE

(30) Priorität: 31.01.2012 EP 12153202
(43) Veröffentlichungstag der Anmeldung: 10.12.2014
(73) Patentinhaber: Covestro Deutschland AG, 51373 Leverkusen (DE)
(72) Erfinder: PENNEMANN, Bernd, 51467 Bergisch Gladbach (DE); MÜNNIG, Jürgen, 41564 Kaarst (DE); DIETRICH, Wulf, 50931 Köln (DE); RAUSCH, Andreas, Karl, 41564 Kaarst (DE)
(74) Vertreter: Levpat
(86) Internationale Anmeldenummer: PCT/EP2013/051561
(87) Internationale Veröffentlichungsnummer: WO 2013/113651

(56) Entgegenhaltungen:
- EP-A1- 0 156 199
- DE-A1- 19 636 191
- DE-A1-102009 005 324
- US-A- 4 091 042

## Beschreibung

Die vorliegende Erfindung betrifft ein Verfahren und eine Anlage zur insbesondere kontinuierlichen Herstellung von Nitrobenzol mittels adiabater Nitrierung von Benzol mit Salpetersäure in Gegenwart von Schwefelsäure, in dem im Anschluss an die Nitrierung eine mehrstufige Aufkonzentrierung der Schwefelsäure mittels Erwärmen bei gegenüber Umgebungsdruck reduziertem Druck ausgeführt wird und wobei das Erwärmen durch Ausnutzung der in der adiabaten Nitrierung von Benzol erzeugten Wärme erfolgt.

Verfahren zur Herstellung von Nitrobenzol durch adiabate Nitrierung von Benzol mittels Salpetersäure in Gegenwart von Schwefelsäure sind bereits seit geraumer Zeit bekannt.

Ein derartiges Verfahren wurde in seiner grundlegenden Erscheinungsform beispielsweise bereits 1939 angemeldet und 1941 als US 2,256,999 zum Patent erteilt.

Heutige, grundlegende Ausführungsformen sind beispielsweise in US 4,091,042, US 5,313,009 und US 5,763,697 beschrieben.

Ein weiteres Verfahren zur adiabatischen Nitrierung von Benzol wird in DE 10 2009 005 324 beschrieben.

Den darin beschriebenen, im Wesentlichen adiabaten Verfahren ist gemein, dass die eigentlichen Ausgangsstoffe der Nitrierung - Benzol und Salpetersäure - in Gegenwart großer Überschüsse an Schwefelsäure zur Reaktion gebracht werden.

Weiter wird in allen vorgenannten Verfahren des Standes der Technik die Salpetersäure mit dem Überschuss an Schwefelsäure vorvermischt. Eine damit erhaltene Mischung umfassend im Wesentlichen Schwefelsäure und Salpetersäure wird dort, wie auch im Rahmen der vorliegenden Erfindung als Mischsäure bezeichnet.

Die Reaktion von Benzol mit Salpetersäure zu Nitrobenzol und Wasser ist stark exotherm (ΔH = -117 kJ/mol), und in den vorgenannten Verfahren nimmt der große Überschuss an Schwefelsäure hierbei die bei der Reaktion frei werdende Reaktionsenthalpie in Form von Wärme, wie auch das hierbei gebildete Wasser beinahe quantitativ auf.

Ebenso ist es den vorgenannten Verfahren gemein, dass die im Reaktionsprodukt gespeicherte Wärme aus der Reaktionsenthalpie mindestens teilweise dazu genutzt wird, die Schwefelsäure, die im Zuge der Reaktion stark mit Wasser verdünnt wird, wieder durch Verdampfung des Wassers aufzukonzentrieren.

So wird in der US 4,091,042 das Verfahren in vier in Reihe geschalteten gerührten Kesseln durchgeführt wird, wobei das den vierten Rührkessel verlassende Reaktionsprodukt in eine ebenso kontinuierlich betriebene Phasentrennvorrichtung geleitet wird, in dem die sogenannte, im Wesentlichen wässrige Absäure von dem sogenannten, im wesentlichen organischen Rohnitrobenzol getrennt wird.

Im Zusammenhang mit der vorliegenden Erfindung bezeichnet Absäure ein im Wesentlichen polares, wässriges Gemisch umfassend Wasser und Schwefelsäure, in dem gegebenenfalls in der Schwefelsäure noch Anteile von Nitrobenzol gelöst sind, und Rohnitrobenzol bezeichnet ein im Wesentlichen unpolares, organisches Gemisch umfassend Benzol und Nitrobenzol, in dem gegebenenfalls in dem Nitrobenzol noch Anteile von Schwefelsäure und Wasser gelöst sind.

In der US 4,091,042 wird die Absäure unter Ausnutzung der in der Absäure gespeicherten Reaktionswärme in einem Blitzverdampfer aufkonzentriert. Diese Aufkonzentrierung erfolgt gemäß der US 4,091,042 einstufig bei 90 °C und etwa 80 mbar.

Gemäß der US 4,091,042 wird auch das Rohnitrobenzol weiter aufbereitet, indem es kontinuierlich in eine vierstufige Gegenstromextraktiouswäsche eingeleitet wird, in der saure Bestandteile wie Schwefelsäurereste und Nebenreaktionprodukte, wie z. B. Dinitrophenol und Pikrinsäure durch den Kontakt mit einer Natriumcarbonatlösung extrahiert werden.

Das somit gereinigte Rohnitrobenzol wird abschließend einer Wasserdampfdestillation unterzogen, in der das verbleibende Benzol für Zwecke der Rückführung in die Nitrierung abgetrennt wird.

In der US 4,091,042 wird also keine mehrstufige Verdampfung offenbart.

Auch in dem in der US 5,313,009 beschriebenen Verfahren zur adiabaten Herstellung von Nitrobenzol wird Salpetersäure mit einer großen Menge an Schwefelsäure zur Mischsäure vermischt und in diese Mischsäure wird Benzol dosiert, welches dann mit der Salpetersäure zu Wasser und im Wesentlichen Nitrobenzol reagiert.

Gemäß dem Verfahren nach der US 5,313,009 wird die Temperatur der Reaktion, die Konzentrationen von Benzol, die Konzentration von Salpetersäure, wie auch die Konzentration von Schwefelsäure so gewählt, dass - im Gegensatz zum Verfahren nach der vorgenannten US 4,091,042 - bereits nach einer ersten Reaktionszone eine im Wesentlichen salpetersäurefreie Mischung aus Benzol, Nitrobenzol, Schwefelsäure und Wasser erhalten wird. D. h. gemäß der US 5,313,009 wird das Verfahren so betrieben, dass es zu einem quantitativen Umsatz der Salpetersäure kommt. Hierzu wird Benzol bezogen auf die Salpetersäuremenge mindestens in stöchiometrischen Mengen eingesetzt.

Das im Wesentlichen salpetersäurefreie Reaktionsgemisch, das nach der Reaktionszone erhalten wird, wird auch in der US 5,313,009 einer einzelnen Phasentrennvorrichtung zugeführt, in der das Rohnitrobenzol von der Absäure getrennt werden.

Die Absäure wird analog zu dem Verfahren gemäß der US 4,091,042 einem Blitzverdampfer zugeführt, in dem ebenso bei gegenüber Umgebungsdruck reduziertem Druck und bei erhöhter Temperatur das Wasser aus der Absäure verdampft wird. Auch hier wird die Wärme aus den Reaktionspodukten wenigstens teilweise für die Aufkonzentrierung der Schwefelsäure in genanntem Blitzverdampfer eingesetzt.

Die US 5,313,009 beschreibt lediglich, dass die aus der Blitzverdampfung erhaltene konzentrierte Säure in ihrer Zusammensetzung etwa jener identisch ist, wie sie eingangs des Verfahrens eingesetzt wurde. Den genauen Druck der Blitzverdampfung, wie auch die genaue Temperatur der Blitzverdampfung offenbart die US 5,313,009 nicht.

Die US 5,313,009 offenbart somit ebenso wenig eine mehrstufige Blitzverdampfung der Absäure.

Auch in dem Verfahren gemäß der US 5,763,697 wird nur von einer "Reinigungs- und Aufkonzentrierungstufe gesprochen", wobei aber die genaue Ausführung dieser Reinigung und Aufkonzentrierung in der US 5,763,697 nicht offenbart wird. Im Übrigen ähnelt das Verfahren der US 5,763,697 jenem der vorbeschriebenen US 5,313,009.

Die vorstehenden, allgemein bekannten Verfahren des Standes der Technik, sind mithin durchgängig dadurch gekennzeichnet, dass die weitere Behandlung der Absäure einstufig in einer Blitzverdampfung unter verringertem Druck erfolgt, deren Wärme zumindest teilweise aus der adiabaten Reaktion von Benzol mit der Salpetersäure stammt.

Hieraus resultiert, dass im Allgemeinen neben der erwünschten Verdampfung des Wassers zur Aufkonzentrierung der Schwefelsäure auch weitere Bestandteile der Absäure mit verdampft werden, weil in einer Stufe die erwünschte Abtrennrate des Wassers erreicht werden muss, was bei durch die Reaktionsenthalpie vorgegebener, zur Verfügung stehender Wärme alleine durch die starke und insbesondere schnelle Druckerniedrigung erreicht werden kann.

Somit kommt es neben der Verdampfung von Wasser z. B. auch zur Ausgasung von Stickoxiden die als Produkte von Nebenreaktionen wie z. B. Oxidationen während der Nitrierreaktion entstehen. Stickstoffmonoxid und Distickstoffmonoxid weisen hierbei Siedepunkte von etwa -150°C bzw. -90 °C auf, so dass diese im Weiteren nicht durch Kondensation zurück gewonnen werden können.

Neben diesen bilden sich auch weitere im Folgenden nicht kondensierbare Gase genannte Produkte durch die einfache Blitzverdampfung, welche auf Grund ihres geringen Siedepunktes nicht mehr wirtschaftlich zurückgewonnen werden können. Hieraus resultieren insbesondere Nachteile bezogen auf die Dimensionierung der Kondensationsvorrichtungen und Vorrichtungen für die Erzeugung des benötigten Unterdrucks. In beiden Fällen muss wegen der signifikant erhöhten Volumenströme auf größer dimensionierten Vorrichtungen zurückgegriffen werden, was nicht zu Letzt die Rentabilität solcher Verfahren negativ beeinflusst.

Den vorgenannten Problemen widmet sich mit Blick auf besondere, die Belastung der Umwelt betreffende Einzelproblemstellungen die DE 19636191. Diese beschreibt im Unterschied zum vorgenannten Stand der Technik ein mehrstufiges Reinigungsverfahren für die Absäure. Das mehrstufige Verfahren gemäß der DE 19636191 umfasst einen ersten Schritt, in dem sogenannte wasserdampfflüchtige organische Verbindungen durch Überleiten von Wasserdampf ("Wasserdampfstrippen") bei einem Druck im Bereich von 200 mbar bis 1000 mbar im Gegenstrom entfernt werden, woraufhin in einem zweiten Schritt bei identischem Druck eine erste Verdampfung des Wassers aus der verbleibenden Schwefelsäure ausgeführt wird und in einem dritten Schritt mehrfach, bei weiter erniedrigtem Druck die Verdampfung des Wassers fortgeführt wird.

Im Gegensatz zu den Verfahren aus dem vorherigen Stand der Technik wird bei der Verdampfung des Wassers die Wärme von außen und aus anderer Quelle zugeführt. Eine Nutzung der Wärme aus der Nitrierung zur Verdampfung des Wassers aus der Absäure wird nicht offenbart.

Auch die EP 0615951 beschreibt eine mehrstufige Verdampfung des Wassers aus der Absäure, der analog der DE 19636191 eine zusätzliche Reinigungsstufe in Form eines Wasserdampfstrippens vorgeschaltet ist. Wie auch die DE 19636191 offenbart die EP 0615951 keine Verwendung der Wärme aus der adiabaten Nitrierung.

Der in EP 0615951 und DE 19636191 beschriebene Ansatz ist zusammengefasst auch Ullmann ("Sulfuric acid und sulfur trioxide", 2005, Wiley VCH, S. 51 bis 52) zu entnehmen. Hier wird beschrieben, dass eine mehrstufige Aufkonzentrierung der Schwefelsäure nach Lurgi, (Fig. 42), bei der der Druck von 1 bar nach 70 mbar abnimmt, um ein Temperaturgefälle zu erzeugen, eine Einsparung von Energie ermöglicht. In den dort vorgestellten Varianten wird jeweils die Kondensationswärme aus der Rekondensation des Dampfes einer Verdampfungsstufe zum Beheizen der vorherigen Stufe benutzt.

Eine mehrstufige Eindampfung von Schwefelsäure wie in DE 19636191, EP 0615951 und Ullmann (2005) beschrieben, ist aus energetischen Gesichtspunkten aber nur dann sinnvoll, wenn der Konzentrationsunterschied der Absäure zwischen den verschiedenen Stufen der Aufkonzentrierung hinreichend groß ist, so dass die Kondensationswärme aus Rekondensation des Dampfes der vorherigen Verdampfungsstufe ("Brüden") zum Beheizen der folgenden Stufe benutzt werden kann.

Bei der Aufkonzentrierung der Absäure aus der Nitrierung von Benzol ist dies im Allgemeinen nicht der Fall.

Die technischen Lehren der DE 19636191 und der EP 0615951 beziehen sich insbesondere auf die Behandlung von Absäuren aus der Nitrierung von Toluolen, welche sodann aus den Absäuren abzutrennen sind. Im Gegensatz zu den in der vorliegenden Erfindung betroffenen nitrierten Benzolen weisen diese Mono- und/oder Di-Nitrotoluole aber keine geeigneten Taupunkte bzw. bei den Taupunkten geeigneten Konzentrationsunterschiede auf, so dass eine Übertragung der darin enthaltenen technischen Lehren auf das Problem der Herstellung von Nitrobenzol und der darin enthaltenen Problemstellung der Abtrennung und Aufkonzentrierung der Schwefelsäure mit diesen Verunreinigungen nicht möglich ist.

Es besteht also nach wie vor die Aufgabe ein Verfahren zur Verfügung zu stellen, das die Herstellung von Nitrobenzol durch Nitrierung von Benzol mit Salpetersäure in Gegenwart von Schwefelsäure ermöglicht und dabei die genannten Nachteile des Standes der Technik bezogen auf die Herstellung von Nitrobenzol nicht aufweist.

Insbesondere soll das Verfahren die Nachteile, die mit der Bildung erheblicher Mengen in der Säure gelöster, nicht kondensierbarer Gase verbunden sind - die wesentlich erhöhten Energieaufwände für deren Abführung bei einstufiger Behandlung - nicht aufweisen und es daher ermöglichen, dass die Vorrichtungen zur Erzeugung von Unterdruck kleiner und damit kostengünstige dimensioniert werden können.

Ein weiteres Problem, dass im Stand der Technik auftritt und bis dato nicht gelöst ist, ist, dass durch die sehr niedrigen Drücke in den Blitzverdampfern des Standes der Technik der Taupunkt des erzeugten Dampfstromes (Brüden) häufig nahe an der Vorlauftemperatur des Kühlmediums üblicher Kühlsysteme (Wasser) liegt, so dass große Wärmetauscherflächen zur Kondensation dieses Stromes erforderlich sind. Infolge der Nähe des Prozessdrucks zum Taupunkt des Wassers besteht ein erheblicher Teil des Gasstroms aus Wasserdampf, wodurch die Kapazitätsanforderungen an die Vakuumpumpe noch weiter steigen.

Insgesamt besteht also nach dem Stand der Technik das Problem ein Verfahren zur Herstellung von Nitrobenzol durch Nitrierung von Benzol mit Salpetersäure in Gegenwart von Schwefelsäure zur Verfügung zu stellen, das es ermöglicht, die Investitionskosten in die Anlage zur Ausführung des Verfahrens signifikant zu senken.

Es wurde nun überraschend gefunden, dass ein
Verfahren zur Herstellung von Nitrobenzol mittels adiabater Nitrierung von Benzol mit Salpetersäure in Gegenwart von Schwefelsäure in dem
a) das in der Nitrierung erhaltene Produktgemisch in Absäure und Rohnitrobenzol getrennt wird und
b) mindestens das in der Absäure enthaltene Wasser von der darin enthaltenen Schwefelsäure teilweise abgetrennt wird und
c) die so aufkonzentrierte Schwefelsäure wieder der Nitrierung zugeführt wird,
dass dadurch gekennzeichnet ist,
i) dass das Abtrennen gemäß dem Schritt b) durch Verdampfen in zwei Stufen mit von erster Stufe zu zweiter Stufe von 100 mbar bis 300 mbar auf 50 mbar bis 150 mbar sinkenden absolutem Druck ausgeführt wird,
ii) dass die für das Verdampfen gemäß Schritt i) eingesetzte Wärme zu einem Anteil von mindestens 90% aus der Reaktionsenthalphie der Nitrierung stammt und
iii) dass das in der Nitrierung erhaltene Produktgemisch eine Temperatur von mindestens 110 °C aufweist,
die vorgenannte Aufgabe zu lösen vermag.

In der ersten Stufe des Schrittes i) des erfindungsgemäßen Verfahrens erfolgt also die Verdampfung von in der Schwefelsäure enthaltendem Wasser bei einem absoluten Druck von 100 mbar bis 300 mbar. In der zweiten Stufe des Schrittes i) des erfindungsgemäßen Verfahrens erfolgt die Verdampfung von in der Schwefelsäure enthaltendem Wasser bei einem absoluten Druck von 50 mbar bis 150 mbar, wobei der Druck in der zweiten Stufe natürlich stets geringer ist als in der ersten Stufe.

Das erfindungsgemäße Abtrennen durch Verdampfen bei den vorgenannten, erfindungsgemäßen Drücken ergibt eine Reihe von Vorteilen, insbesondere wenn die Wärme aus der Reaktionsenthalphie der Nitrierung genutzt wird.

So wird in der ersten Stufe, bei einem vergleichsweise geringfügig erniedrigten Druck durch Verdampfen abgetrennt, wodurch nur hier das Problem des Ausgasens der nicht kondensierbaren Gase auftritt und mithin die erforderliche Kapazität und Nennleistung der Vorrichtung zur Druckverringerung (Vakuumanlage) insgesamt verringert wird.

Insbesondere ist energetische Aufwand in der ersten Stufe geringer, weil hier bei vergleichsweise höheren Drücken (d. h. geringer erniedrigten) gearbeitet werden kann, während in der zweiten Stufe der Druck auf das abschließend gewünschte Maß verringert wird, wobei aber hier der Volumenstrom der Gase durch die vorherige Abtrennung der nicht kondensierbaren Gase verringert ist, so dass auch hier der energetische Aufwand verringert ist. Das Zusammenspiel der beiden Stufen ergibt somit einmal aus der Tatsache eines höheren Drucks in der ersten Stufe und aus der Tatsache eines verringerten Volumenstroms in der zweiten Stufe einen insgesamt deutlich verringerten Energieaufwand des Gesamtverfahrens, der über die Summe der eingesparten Energie einer jeweiligen Einzelmaßnahme (nur erhöhter Druck bei Abtrennung, oder nur verringerter Volumenstrom) hinaus geht. Es hat sich gezeigt, dass dieses Verfahren dann vorteilhaft ist, wenn der Druck in der ersten Stufe mindestens 10 mbar, bevorzugt 30 mbar höher als in der zweiten Stufe ist.

Aus dem vorgenannten Abtrennen bei vergleichsweise hohem Druck resultiert außerdem, dass der vorgenannte Kondensator kleiner dimensioniert werden kann oder dass alternativ geringere Mengen Kühlwassers verwendet werden, da die nicht kondensierbaren Gase nicht mitabgekühlt werden müssen. Außerdem steigt durch den höheren Druck in der ersten Stufe der Taupunkt des erzeugten Brüdenstroms deutlich an, so dass auch in warmen Klimazonen die Kondensation mit Kühlturmwasser erfolgen kann und im Vergleich zu einer Kondensation gegen Kaltwasser eine deutliche Verbesserung der Energieeffizienz erreicht wird. Als Kondensatoren sind alle dem Fachmann bekannten Typen geeignet. So kann die Kondensation direkt oder indirekt, sowohl einwie mehrstufig erfolgen. Geeignet sind beispielsweise Rohrbündelwärmetauscher, ggf. mit Nachkühler.

Insgesamt führt das erfindungsgemäße Verfahren umfassend die Herstellung von Nitrobenzol durch adiabate Nitrierung von Benzol mit anschließender zweistufiger Ausführung der Verdampfung zur Aufkonzentrierung der Absäure zu einer höheren Anlagenverfügbarkeit, niedrigeren Instandhaltungskosten, niedrigeren Investitionskosten und geringeren Energiekosten.

Die vorstehenden Vorteile des erfindungsgemäßen Verfahrens sind im Lichte des Standes der Technik, wie vorstehend diskutiert, überraschend, da im Stand der Technik entweder eine einstufige Verdampfung offenbart wird oder aber eine mehrstufige, die sich aber auf Stoffsysteme bezieht, die die besondere Problemstellung der Nitrobenzole nicht aufweist.

Im vorliegenden Fall der Mononitrierung von Benzol ist wie vorstehend erwähnt der erforderliche Konzentrations- und Siedetemperatureunterschied nicht vorhanden, um die im Stand der Technik beschriebene Nutzung der Kondensationswärme zu erreichen, so dass diese Vorgehensweise nicht in geeigneter Weise auf die bereits im Zusammenhang mit Nitrobenzol beschriebene einstufige Verfahrensweise angewendet werden kann.

In dem erfindungsgemäßen Verfahren weist das in der Nitrierung erhaltene Produktgemisch eine Temperatur von mindestens 110 °C auf. Somit weist auch die Absäure, die durch Abtrennen des Rohnitrobenzols erhalten wird ebenso etwa diese Temperatur auf.

Bevorzugt beträgt die Temperatur des in der Nitrierung erhaltenen Produktgemisches und somit die Temperatur der Absäure mindestens 120 °C.

Bevorzugt beträgt die Temperatur des in der Nitrierung erhaltenen Produktgemisches und somit die Temperatur der Absäure maximal 150 °C.

Die Einhaltung der vorgenannte Mindestemperatur von 110 °C, bevorzugt 120 °C, stellt sicher, dass ausreichend Energie in Form von Wärme im nachfolgenden zweistufigen Abtrennen durch Verdampfen vorhanden ist, um auch bei den weniger verringerten Drücken eine ausreichende Trennleistung vom Wasser zu erzielen.

Die Absäure wird durch Abtrennen mittels Verdampfen von insbesondere Wasser in zwei Stufen aufkonzentriert.

Erfindungsgemäß stammt die Wärme für das Verdampfen zu einem Anteil von mindestens 90 %, bevorzugt 95 % aus der adiabaten Nitrierungsreaktion, indem das Reaktionsprodukt in sich eine ausreichende Enthalpie zum Erreichen dieses Wertes beinhaltet.

Besonders bevorzugt wird in den beiden Stufen des Schrittes i) des erfindungsgemäßen Verfahrens keine weitere Wärme der Absäure zugeführt. Es folgt somit weiter, dass die Verdampfer ebenso adiabat betrieben werden.

In einer ebenso bevorzugten Ausführungsform des vorliegenden erfindungsgemäßen Verfahrens wird ein Anteil von bis zu 10 % der für die Verdampfung eingesetzten Wärme den beiden Stufen von außerhalb des Verfahrens zugeführt.

Dabei können einer oder beide Verdampfer zusätzlich mit Heizdampf oder einer anderen externen Wärmezufuhr ausgerüstet sein.

Diese Ausführungsform ist deshalb bevorzugt, weil sie es ermöglicht, regelnd in die Verdampfung einzugreifen, was andernfalls nur über Druckanpassungen möglich wäre, die aber im Ansprechverhalten langsamer sind, als eine unmittelbare Temperaturanpassung in der jeweiligen Stufe der Verdampfung.

Demgemäß ist das vorliegende erfindungsgemäße Verfahren für seine technische Vorteilhaftigkeit in Bezug auf die vorgenannte Energieeinsparung nicht auf die weitere Zufuhr von Wärme in die beiden Stufen der Verdampfung angewiesen, sondern nutzt diese in bevorzugter Art und Weise alleine für eine bessere verfahrenstechnische Kontrolle des Gesamtverfahrens.

Der Zulauf an Absäure zur ersten Stufe gemäß i) enthält bevorzugt 60 bis 75 Massen-%, besonders bevorzugt 65 bis 70 Massen-% an H₂SO₄.

In der Absäure können neben Wasser und Schwefelsäure auch noch Nitrobenzol, Benzol und die Nebenprodukte der Benzolnitrierung (Dinitrobenzol, Nitrophenole und Oxalsäure) enthalten sein. Auch Salpetersäure sowie gelöste nitrose Gase können enthalten sein.

Der abgetrennte Dampf einer jeden Stufe gemäß i) wird bevorzugt durch ein- oder mehrstufige Kondensation verflüssigt und einer Phasentrennvorrichtung zugeführt, um gleichfalls kondensierte organischen Flüssigkeit abzutrennen.

Damit die aufkonzentrierte Schwefelsäure sinnvoll in die Reaktion zurückgeführt werden kann, sollte diese nach Durchführung beider Stufen des Schritts i) einen Massenanteil an H₂SO₄ von nicht weniger als 65 %, bevorzugt nicht weniger als 68 %, besonders bevorzugt zwischen 69 % und 72 % aufweisen.

Weiterer Gegenstand der vorliegenden Erfindung ist eine Anlage zur Herstellung von Nitrobenzol, bestehend aus einem Reaktor (1), einer Phasentrennvorrichtung (2), zwei Verdampfer- und Kondensationsvorrichtungen verbunden mit Vakuumanlagen (7, 9) und gegebenenfalls einem Lagerbehälter (5), dadurch gekennzeichnet, dass die Anlage einen ersten Verdampfer (3) mit zugehörigem ersten Kondensator (8) und angeschlossener Vakuumanlage (9) und einen zweiten Verdampfer (4) mit zugehörigem zweiten Kondensator (6) und angeschlossener Vakuumanlage (7) aufweist und wobei der erste Verdampfer (3) direkt an den zweiten Verdampfer (4) zur Überleitung nicht verdampfter Anteile angeschlossen ist und wobei ferner die Wärmeaustauschfläche des ersten Kondensators (8) kleiner oder gleich zweimal der Wärmeaustauschfläche des zweiten Kondensators (6) ist.

Die erfindungsgemäße Anlage ist besonders vorteilhaft, weil durch die Anwesenheit der beiden vorgenannten Verdampfer- und Kondensationsvorrichtungen verbunden mit Vakuumanlagen (7, 9) in unmittelbarer Verbindung und durch das erfindungsgemäße Verhältnis der Wärmeaustauschflächen insgesamt gewährleistet wird, dass die Gesamtwärmeaustauschfläche für die in der Anlage unter anderem erzielte Aufkonzentrierung der vorgenannten Absäure verringert werden kann.

Hierdurch werden die Investitionskosten in eine solche Anlage trotz des Vorsehens weiterer - gegenüber dem Stand der Technik - Anlagenteile in Form des zweiten Verdampfers, bzw. des zweiten Kondensators (6) und der zweiten Vakuumanlage (7) signifikant verringert. Dies geht einher mit der Möglichkeit in einer solchen erfindungsgemäßen Anlage Vakuumanlagen einzusetzen, welche insgesamt geringer dimensioniert sind, als eine einzelne, wie sie im Stand der Technik Einsatz findet. Auch dies mindert weiter die Investitionskosten in eine solche Anlage. Gleichsam ist die vorgenannte erfindungsgemäße Anlage nicht nur hinsichtlich ihrer Investitionskosten gegenüber dem Stand der Technik vorteilhaft, sondern führt im Zusammenhang mit dem hier offenbarten, erfindungsgemäßen Verfahren auch dazu, dass die Betriebskosten der Anlage gegenüber dem Stand der Technik verringert sind, wobei aber die Wiederverwendbarkeit der im Verfahren eingesetzten Schwefelsäure hierdurch nicht negativ beeinträchtigt wird.

Üblicherweise sind alle Teile der Anlage, welche unmittelbar mit flüssigen Bestandteilen der in der Anlage behandelten Stoffe in Kontakt gelangen entweder an ihren Innenwänden emailliert oder bestehen aus Tantal, oder ähnlichen - gegenüber heißer Schwefel- und Salpetersäure beständigen - Werkstoffen.

In einer weiteren Ausführungsform der erfindungsgemäßen Anlage sind die Vakuumanlagen (7, 9) so angeordnet, dass die zweite Vakuumanlage (7), welche an den zweiten Kondensator (6) angeschlossen ist, in den ersten Kondensator (8) hineinfördert und wobei die erste Vakuumanlage (9) nach wie vor aus dem ersten Kondensator (8) absaugt. Hierdurch kann die zweite Vakuumanlage (7) geringer dimensioniert werden, da diese nicht mehr gegen den Umgebungsdruck fördert, sondern gegen den bereits durch die erste Vakuumanlage (9) gemäß dem erfindungsgemäßen Verfahren auf 100 mbar bis 300 mbar verringerten Druck. Dies führt zu einer weiteren Einsparung an Energie und Investitionskosten.

Das erfindungsgemäße Verfahren wird nachfolgend anhand von Abbildungen und Beispielen näher erläutert, ohne es jedoch hierdurch hierauf zu beschränken.

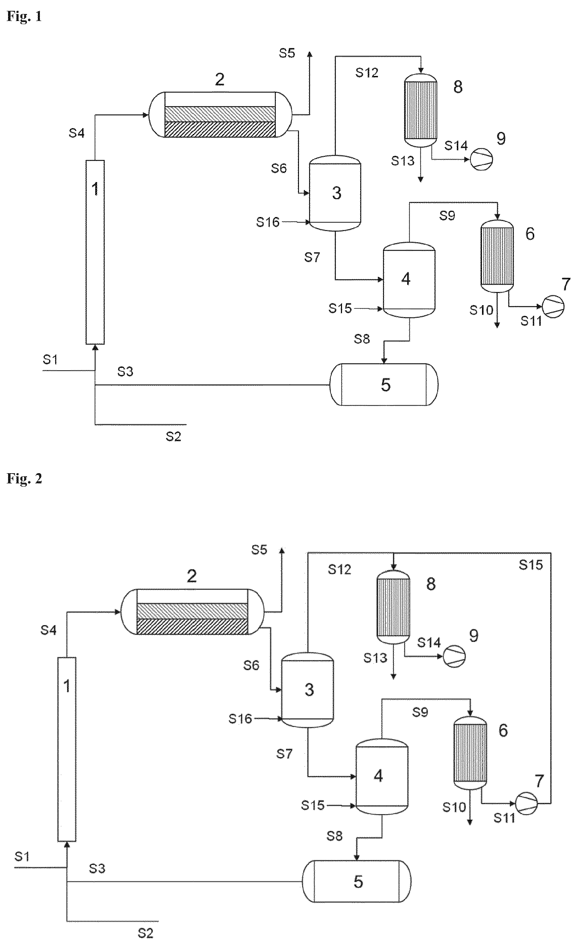

Fig. 1 zeigt schematisch eine bevorzugte Ausführungsform des erfindungsgemäßen Verfahrens bzw. der erfindungsgemäßen Anlage, in dem einem Reaktor (1) ein Schwefelsäurestrom (S3), sowie Salpetersäure (S2) und Benzol (S1) zugeführt werden.

Nach vollständigem Umsatz der Salpetersäure mit Benzol zu Nitrobenzol und Wasser unter adiabaten Bedingungen im Reaktor (1) wird das hierdurch auf etwa 135 °C (vgl. die Beispiele) erwärmte Reaktionsgemisch (S4) einer Phasentrennvorrichtung in Form eines Dekanters (2) zugeführt, in dem das Reaktionsgemisch in eine Phase von Rohnitrobenzol (S5) und Absäure (S6) aufgetrennt wird. Die Absäure (S6) wird in einem ersten bei 260 mbar betriebenen Verdampfer (3) teilweise aufkonzentriert. Ebenfalls dargestellt ist ein Falschluftstrom (S16), der durch das Vakuum in dem ersten Verdampfer (3) mit in diesen eingesogen wird. Der im ersten Verdampfer (3) erzeugte Dampfstrom (S12) umfassend Wasser, Schwefelsäure, Nitrobenzol, Benzol sowie aliphatische organische Verbindungen, die als Verunreinigungen in dem Benzolstrom (S1) zur Reaktion enthalten sind, werden in einem Kondensator (8) zu einem ersten flüssigen Ablaufstrom (S13) kondensiert. Der nicht verdampfte, flüssige Ablaufstrom des ersten Verdampfers (S7) wird dem zweiten, bei einem Druck von 74 mbar betriebenen Verdampfer (4) zugeführt und hierdurch auf die eine Konzentration von 70 Massen-% Schwefelsäure (S8) aufkonzentriert. Ebenfalls dargestellt ist ein zweiter Falschluftstrom (S 15), der durch das Vakuum in dem zweiten Verdampfer (3) mit in diesen eingesogen wird. Für die weitere Verwendung wird die somit wieder aufkonzentrierte Schwefelsäure (S8) in einem Schwefelsäuretank (5) gelagert. Bei der zweiten Verdampfung der Absäure wird ein Dampfstrom (S9) bestehend im Wesentlichen aus Wasser, sowie Resten an Schwefelsäure, Nitrobenzol und Benzol erhalten, der in einem Kondensator (6) kondensiert wird.

Ein großer Teil der in der Absäure (S6) gelösten nicht kondensierbaren Gase geht in dem ersten Verdampfer (3) in die Dampfphase (S12) über und wird als nicht kondensierbare Dampfphase (S14) über das Vakuumanlage (9) des ersten Kondensators (8) abgeführt. Der größte Teil der in der Absäure gelösten organischen Komponenten die leichter als Wasser sieden, gehen ebenfalls in dem ersten Verdampfer (3) in die Dampfphase (S12) über und werden im Kondensator (8) zum Strom (S13) kondensiert. Im zweiten Verdampfer (4) besteht die Dampfphase (S9) im Wesentlichen aus Wasser und Nitrobenzol, so dass der Taupunkt im Kondensator (6) durch die Komponente Wasser bestimmt wird, welches als flüssiger Strom (S10) aus dem Verfahren herausgeführt werden kann. Verbleibende nicht kondensierbare Gase (S11) werden über die Vakuumanlage des zweiten Kondensators (7) abgeführt.

Der Taupunkt der Dampfphase (S9) liegt bei vergleichbarem Druck höher als jener der Dampfphase (S12), der aus einer einstufigen Aufkonzentrierung der Schwefelsäure resultieren würde.

Fig. 2 zeigt eine weitere, besonders bevorzugte Ausführungsform des erfindungsgemäßen Verfahrens bzw. der erfindungsgemäßen Anlage, die im Wesentlichen identisch ist mit jener gemäß Fig. 1, sich aber dadurch unterscheidet, dass aus der zweiten Vakuumanlage (7) der Gasstrom (S15) in den ersten Kondensator (8) geführt wird, so dass dieser Gasstrom (S15) und die Dampfphase (S12) aus dem ersten Verdampfer (3) zusammen in den ersten Kondensator (8) geführt werden. Dies führt zu einer kleiner dimensionierten zweiten Vakuumanlage (7).

### Beispiele

### Beispiel 1 (Vergleichsbeispiel): Einstufige Verdampfung

Mit Hilfe eines Prozessmodells wurde die Aufkonzentrierung der verdünnten Schwefelsäure durch einstufige Verdampfung simuliert. Die Prozessparameter und Mengenströme für diese Simulationsrechnung wurden dem Beispiel in US 2001 0043894 A1 entnommen.

Diese Verfahrensweise entspricht jener der Fig. 1, mit dem Unterschied, das die Vorrichtungen (3), (8) und (9) nicht existieren und der Strom (S6) somit direkt in den Verdampfer (4) gelangt. D. h. die Verdampfung erfolgt einstufig nach dem Stand der Technik.

Dem Reaktor (1) wird ein Gemisch bestehend aus einem Säurestrom mit Massenanteilen von 65,7 % H₂SO₄, 4,0 % HNO₃ und 30,3 % H₂O sowie einem Benzolstrom zugeführt. Das Benzol wird dabei in einer Menge eingesetzt, so dass sich ein molares Verhältnis von 1,1 : 1 zwischen Benzol bezogen auf HNO₃ einstellt.

Der Gesamtvolumenstrom durch den Reaktor (1) beträgt 173 m³/h.

Dieser Strom (S4) verlässt den Reaktor (1) bei adiabater Reaktionsführung mit 135°C und wird einer Phasentrennvorrichtung (Dekanter, 2) zugeführt. Bei einem Phasenverhältnis zwischen Benzol- und Säurestrom von 1 : 18,5 nach Gewichtsanteilen am Eintritt in den Reaktor (1) trennt sich der Strom (S4) in der Phasentrennvorrichtung (2) in eine Absäurephase (S6) mit 157 m³/h und eine Rohnitrobenzolphase (S5) mit 16 m³/h.

Die Temperatur der Absäure (S6), die als schwere Phase aus der Phasentrennvorrichtung (2) abläuft, beträgt 135 °C und der Schwefelsäureanteil 67,2 Massen-%.

Um eine Aufkonzentrierung der Schwefelsäure auf 70 Massen-% in Strom (S8) zu erreichen, beträgt im Verdampfer (4) die Temperatur 95 °C und der Druck 74 mbar.

Der entstehende Dampfstrom (S9) im Umfang von 9,3 t/h besteht im Wesentlichen aus Wasserdampf, welcher einem Kondensator (6) zugeführt wird.

Unter Berücksichtigung eines Druckverlusts von 10 mbar zwischen Verdampfer (4) und Kondensator (6) ergibt sich damit ein Kondensationsdruck von 64 mbar.

Unter diesen Bedingungen kann bei einer Kühlwasservorlauftemperatur von 30 °C und einer Temperaturdifferenz von 2 K zwischen Kühlwasserseite und Prozessseite die Dampfphase (S9) bis auf einen Anteil von 0,6 % kondensiert werden.

Hierzu müssen 5,56 MW Kondensationswärme abgeführt werden. Dazu ist ein Kondensator mit einer Wärmeübertragungsfläche von 920 m² erforderlich.

Der nicht kondensierbare Anteil wird als Abgasstrom (S11) über die Vakuumanlage (7) abgeführt.

Der entsprechende Volumenstrom beträgt 850 m³/h. Die erforderliche Leistung einer geeigneten Vakuumanlage (7) zur Förderung dieses Volumenstroms von 850 m³/h bei 64 mbar Saugdruck beträgt etwa 40 kW.

Für das gesamte Vakuumsystem einer einstufigen Verdampfung wurde auf der Basis von betrieblichen Erfahrungswerten ein Falschluftstrom (S15) von 10 kg/h angenommen, der über minimale Undichtigkeiten in Flanschverbindungen von außen in den unter Vakuum betriebenen Anlagenteil gelangt.

### Beispiel 2 (Erfindungsgemäß): Zweistufige Verdampfung

Diese Verfahrensalternative entspricht der Fig. 1. Die Temperatur der Absäure (S6), die als schwere Phase aus dem Phasentrennapparat (2) abläuft, beträgt wiederum 135°C und der Schwefelsäureanteil 67,1 Massen-%.

Die Absäure (S6) wird zunächst dem ersten Verdampfer (3) zugeführt. Im ersten Verdampfer (3) wird die Absäure auf 68,2 Massen-% aufkonzentriert, wobei im Verdampfer (3) ein Druck von 260 mbar und eine Temperatur von 120°C eingestellt sind.

Die entstehende Dampfphase (S12) wird dem Kondensator (8) zugeführt. Unter Berücksichtigung eines zu Beispiel 1 analogen Druckverlusts von 10 mbar zwischen Verdampfer (3) und Kondensator (8) ergibt sich damit ein Kondensationsdruck von 250 mbar.

Unter diesen Bedingungen kann bei einer Kühlwasservorlauftemperatur von 30°C und einer Temperaturdifferenz von 2 K zwischen Kühlwasserseite und Prozessseite die Dampfphase (S12) bis auf einen Anteil von 0,39 % kondensiert werden. Hierfür werden 1,92 MW Kondensationswärme abgeführt. Dazu ist ein Kondensator (8) mit einer Wärmeübertragungsfläche von 82 m² erforderlich.

Der nicht kondensierbare Anteil wird als Abgasstrom (S14) über die Vakuumanlage(9) abgeführt. Der entsprechende Volumenstrom beträgt 4,5 m³/h. Die erforderliche Leistung einer geeigneten Vakuumanlage (9) zur Förderung eines Saugstroms von 4,5 m³/h bei 250 mbar Saugdruck beträgt etwa 1 kW.

Der flüssige Ablauf (S7) aus dem ersten Verdampfer wird einem zweiten Verdampfer (4) zugeführt. Um eine Aufkonzentrierung der Schwefelsäure auf 70 Massen-% in Strom (S8) zu erreichen, wird im zweiten Verdampfer (4) eine Temperatur von 95°C und ein Druck 74 mbar eingestellt.

Die entstehende Dampfphase (S9) wird einem weiteren Kondensator (6) zugeführt. Unter Berücksichtigung eines Druckverlusts von 10 mbar zwischen Verdampfer (4) und Kondensator (6) ergibt sich damit ein Kondensationsdruck von 64 mbar.

Unter diesen Bedingungen kann bei einer Kühlwasservorlauftemperatur von 30°C und einer Temperaturdifferenz von 2 K zwischen Kühlwasserseite und Prozessseite die Dampfphase (S9) bis auf einen Anteil von 0,54 % kondensiert werden.

Dazu müssen 3,64 MW Kondensationswärme abgeführt werden. Dazu ist ein Kondensator (6) mit einer Wärmeübertragungsfläche von 640 m² erforderlich.

Der nicht kondensierbare Anteil wird als Abgasstrom (S11) über die Vakuumpumpe (7) abgeführt. Der entsprechende Volumenstrom beträgt 405 m³/h. Die erforderliche Leistung einer geeigneten Vakuumanlage zur Förderung eines Saugstroms von 405m³/h bei 64 mbar Saugdruck beträgt etwa 25 kW.

Das Verhältnis der Massenströme von Dampfphase (S12) aus dem ersten Verdampfer (3) und Dampfphase (S9) aus dem zweiten Verdampfer (4) beträgt in diesem Beispiel 1 : 1,6.

Im Vergleich zum Verfahren nach dem Stand der Technik (Beispiel 1) kann damit die erforderliche Wärmeübertragerfläche zur Kondensation der Brüdenströme durch den Einsatz einer zweistufigen Verdampfung von 920 m² um 22 % auf 722 m² reduziert werden.

Zusätzlich sinkt die erforderliche Gesamtleistung der Vakuumanlagen von 40 kW um 35% auf 26 kW.

Für das gesamte Vakuumsystem umfassend die beiden Vakuumanlagen (z. B. zwei Kompressoren oder Vakuumpumpen) einer zweistufigen Verdampfung wurde ebenso auf Basis betrieblicher Erfahrungswerte eine Leckrate (sog. "Falschluftstrom") (S15, S16) von 10 kg/h je Verdampfer, d. h. insgesamt 20 kg/h angenommen, der über minimale Undichtigkeiten in Flanschverbindungen von außen in den unter Vakuum betriebenen Anlagenteil gelangt.

D. h. das erfindungsgemäße Verfahren ist auch bei größeren "Falschluftströmen" immer noch vorteilhaft gegenüber Verfahren aus dem Stand der Technik.

### Beispiel 3 (Erfindungsgemäß): Zweistufige Verdampfung

Diese Verfahrensalternative entspricht der Abbildung 1. Die Temperatur der Absäure (S6), die als schwere Phase aus dem Phasentrennapparat (2) abläuft, beträgt wiederum 135°C und der Schwefelsäureanteil 67,1 Massen-%.

Die Absäure (S6) wird zunächst dem ersten Verdampfer (3) zugeführt. Im ersten Verdampfer (3) wird die Absäure auf 69,0 Massen-% aufkonzentriert, wobei sich im Verdampfer (3) ein Druck von 135 mbar und eine Temperatur von 106°C einstellen.

Die entstehende Dampfphase (S12) wird dem Kondensator (8) zugeführt. Unter Berücksichtigung eines zu Beispiel 1 analogen Druckverlusts von 10 mbar zwischen Verdampfer (3) und Kondensator (8) ergibt sich damit ein Kondensationsdruck von 125 mbar. Unter diesen Bedingungen kann bei einer Kühlwasservorlauftemperatur von 30°C und einer Temperaturdifferenz von 2K zwischen Kühlwasserseite und Prozessseite die Dampfphase (S12) bis auf einen Anteil von 0,30% kondensiert werden. Dazu müssen 3,56 MW Kondensationswärme abgeführt werden. Dazu ist ein Kondensator (8) mit einer Wärmeübertragungsfläche von 350 m² erforderlich.

Der nicht kondensierbare Anteil muss als Abgasstrom (S14) zur Vakuumpumpe (9) abgeführt werden. Der entsprechende Volumenstrom beträgt 140 m³/h. Die erforderliche Leistung einer geeigneten Vakuumpumpe zur Förderung eines Saugstroms von 140 m³/h bei 125 mbar Saugdruck beträgt 6 kW.

Der flüssige Ablauf (S7) aus dem ersten Verdampfer wird einem zweiten Verdampfer (4) zugeführt. Um eine Aufkonzentrierung der Schwefelsäure auf 70 Massen-% in Strom (S8) zu erreichen, muss im zweiten Verdampfer (4) bei einer Temperatur von 95°C der Druck 74 mbar betragen.

Die entstehende Dampfphase (S9) wird dem Kondensator (6) zugeführt. Unter Berücksichtigung eines Druckverlusts von 10 mbar zwischen Verdampfer (4) und Kondensator (6) ergibt sich damit ein Kondensationsdruck von 64 mbar. Unter diesen Bedingungen kann bei einer Kühlwasservorlauftemperatur von 30°C und einer Temperaturdifferenz von 2K zwischen Kühlwasserseite und Prozessseite die Dampfphase (S9) bis auf einen Anteil von 1% kondensiert werden. Dazu müssen 1,98 MW Kondensationswärme abgeführt werden. Dazu ist ein Kondensator (6) mit einer Wärmeübertragungsfläche von 320 m² erforderlich. Der nicht kondensierbare Anteil muss als Abgasstrom (S11) zur Vakuumpumpe (7) abgeführt werden. Der entsprechende Volumenstrom beträgt 430 m³/h. Die erforderliche Leistung einer geeigneten Vakuumpumpe zur Förderung eines Saugstroms von 430 m³/h bei 64 mbar Saugdruck beträgt ca. 27 kW. Das Verhältnis der Massenströme von Dampfphase (S12) aus dem ersten Verdampfer und Dampfphase (S9) aus dem zweiten Verdampfer beträgt in diesem Beispiel 2:1.

Im Vergleich zu Beispiel 1 kann damit die erforderliche Wänneüberträgerfläche zur Kondensation der Brüdenströme durch den Einsatz einer zweistufigen Verdampfung von 920 m² um 28% auf 670 m² reduziert werden. Zusätzlich sinkt die erforderliche Leistung der Vakuumpumpen von 40 kW um 18% auf 33 kW. Für das gesamte Vakuumsystem einer zweistufigen Verdampfung wurde auf der Basis von betrieblichen Erfahrungswerten ein Falschluftstrom (S15) von 10 kg/h je Verdampfer, d.h. insgesamt 20 kg/h angenommen, der über minimale Undichtigkeiten in Flanschverbindungen von außen in den unter Vakuum betriebenen Anlagenteil gelangt.

Im Vergleich zu Beispiel 2 ist die erforderliche Wärmeüberträgerfläche zur Kondensation der Brüdenströme in Beispiel 3 um 52 m² auf einen Wert von 670 m² reduziert, die erforderliche Leistung der Vakuumpumpen dagegen um 7 kW auf einen Wert von 33 kW erhöht. Ob eine Ausgestaltung des erfindungsgemäßen Verfahrens gemäß Beispiel 2 oder Beispiel 3 vorzuziehen ist, hängt von den Randbedingungen (z. B. Energiepreisentwicklung) ab.

## Patentansprüche

1. Verfahren zur Herstellung von Nitrobenzol mittels adiabater Nitrierung von Benzol mit Salpetersäure in Gegenwart von Schwefelsäure in dem
a) das in der Nitrierung erhaltene Produktgemisch in Absäure und Rohnitrobenzol getrennt wird und
b) mindestens das in der Absäure enthaltene Wasser von der darin enthaltenen Schwefelsäure teilweise abgetrennt wird und
c) die so aufkonzentrierte Schwefelsäure wieder der Nitrierung zugeführt wird,
dass **dadurch gekennzeichnet ist,**
i) **dass** das Abtrennen gemäß dem Schritt b) durch Verdampfen in zwei Stufen mit von erster Stufe zu zweiter Stufe von 100 mbar bis 300 mbar auf 50 mbar bis 150 mbar sinkenden absolutem Druck ausgeführt wird,
ii) **dass** die für das Verdampfen gemäß Schritt i) eingesetzte Wärme zu einem Anteil von mindestens 90 % aus der Reaktionsenthalphie der Nitrierung stammt und
iii) **dass** das in der Nitrierung erhaltene Produktgemisch eine Temperatur von mindestens 110°C aufweist

2. Verfahren gemäß Anspruch 1, **dadurch gekennzeichnet, dass** das in der Nitrierung erhaltene Produktgemisch eine Temperatur von mindestens 120 °C aufweist.

3. Verfahren gemäß Anspruch 1 oder 2, **dadurch gekennzeichnet, dass** die Wärme für das Verdampfen gemäß Schritt ii) zu einem Anteil von mindestens 90% aus der Reaktionsenthalphie adiabaten Nitrierungsreaktion stammt.

4. Verfahren gemäß Anspruch 3, **dadurch gekennzeichnet, dass** die Wärme für das Verdampfen gemäß Schritt ii) alleine aus der Reaktionsenthalphie adiabaten Nitrierungsreaktion stammt.

5. Verfahren gemäß Anspruch 3, **dadurch gekennzeichnet, dass** ein Anteil von bis zu 10 % der für die Verdampfung eingesetzten Wärme in den beiden Stufen von außen dem Verfahren zugeführt wird.

6. Verfahren gemäß einem der vorstehenden Ansprüche, **dadurch gekennzeichnet, dass** der Zulauf an Absäure zur ersten Stufe gemäß i) 60 bis 75 Massen-% an H₂SO₄ enthält.

7. Verfahren gemäß einem der vorstehenden Ansprüche, **dadurch gekennzeichnet, dass** der in einer jeden Stufe gemäß i) abgetrennte Dampf durch Kondensation verflüssigt wird.

8. Verfahren gemäß Anspruch 7, **dadurch gekennzeichnet, dass** der abgetrennte, verflüssigte Dampf einer Phasentrennvorrichtung zugeführt wird.

9. Verfahren nach einem der vorstehenden Ansprüche, **dadurch gekennzeichnet, dass** die aufkonzentrierte Schwefelsäure gemäß Schritt c) nach Durchführung beider Stufen des Schritts i) einen Massenanteil H₂SO₄ von nicht weniger als 65 % aufweist.

10. Anlage zur Herstellung von Nitrobenzol, bestehend aus einem Reaktor (1), einer Phasentrennvorrichtung (2), zwei Verdampfer- und Kondensationsvorrichtungen verbunden mit Vakuumanlagen (7, 9) und gegebenenfalls einem Lagerbehälter (5),
**dadurch gekennzeichnet, dass**
die Anlage einen ersten Verdampfer (3) mit zugehörigem ersten Kondensator (8) und angeschlossener Vakuumanlage (9) und einen zweiten Verdampfer (4) mit zugehörigem zweiten Kondensator (6) und angeschlossener Vakuumanlage (7) aufweist und wobei der erste Verdampfer (3) direkt an den zweiten Verdampfer (4) zur Überleitung nicht verdampfter Anteile angeschlossen ist und wobei ferner die Wärmeaustauschfläche des ersten Kondensators (8) kleiner oder gleich zweimal der Wärmeaustauschfläche des zweiten Kondensators (6) ist.

## Claims

1. Process for preparing nitrobenzene by adiabatic nitration of benzene by means of nitric acid in the presence of sulfuric acid, in which
a) the product mixture obtained in the nitration is separated into used acid and crude nitrobenzene and
b) at least the water present in the used acid is partly separated off from the sulfuric acid present therein and
c) the sulfuric acid which has been concentrated in this way is fed back to the nitration,
**characterized**
i) **in that** the separation as per step b) is carried out by evaporation in two stages at an absolute pressure which decreases from the first stage to the second stage from 100 mbar-300 mbarto 50 mbar-150 mbar,
ii) **in that** the heat used for the evaporation as per step i) originates to an extent of at least 90% from the enthalpy of reaction of the nitration and
iii) **in that** the product mixture obtained in the nitration has a temperature of at least 110°C.

2. Process according to Claim 1, **characterized in that** the product mixture obtained in the nitration has a temperature of at least 120°C.

3. Process according to Claim 1 or 2, **characterized in that** the heat for the evaporation as per step ii) originates to an extent of at least 90% from the enthalpy of reaction of the adiabatic nitration reaction.

4. Process according to Claim 3, **characterized in that** the heat for the evaporation as per step ii) originates solely from the enthalpy of reaction of the adiabatic nitration reaction.

5. Process according to Claim 3, **characterized in that** a proportion of up to 10% of the heat used for the evaporation in the two stages is supplied to the process from the outside.

6. Process according to any of the preceding claims, **characterized in that** the feed stream of used acid to the first stage in i) contains from 60 to 75% by mass of H₂SO₄.

7. Process according to any of the preceding claims, **characterized in that** the vapor separated off in each stage in i) is liquefied by condensation.

8. Process according to Claim 7, **characterized in that** the liquefied vapor which has been separated off is fed to a phase separation apparatus.

9. Process according to any of the preceding claims, **characterized in that** the sulfuric acid which has been concentrated in step c) has a proportion by mass of H₂SO₄ of not less than 65% after both stages of step i) have been carried out.

10. Plant for preparing nitrobenzene, which consists of a reactor (1), a phase separation apparatus (2), two evaporation and condensation apparatuses connected to vacuum units (7, 9) and optionally a storage vessel (5),
**characterized in that**
the plant has a first evaporator (3) with associated first condenser (8) and attached vacuum unit (9) and a second evaporator (4) with associated second condenser (6) and attached vacuum unit (7) and **in that** the first evaporator (3) is connected directly to the second evaporator (4) to transfer material which has not been evaporated and **in that** the heat transfer area of the first condenser (8) is smaller than or equal to twice the heat transfer area of the second condenser (6).

## Revendications

1. Procédé pour la préparation de nitrobenzène au moyen d'une nitruration adiabatique de benzène avec de l'acide nitrique en présence d'acide sulfurique, dans lequel
a) le mélange de produits obtenu dans la nitruration est séparé en acide résiduaire et en nitrobenzène brut et
b) au moins l'eau contenue dans l'acide résiduaire est séparée partiellement de l'acide sulfurique qui y est contenu et
c) l'acide sulfurique ainsi concentré est de nouveau introduit dans la nitruration,
**caractérisé en ce que**
i) la séparation selon l'étape b) est réalisée par évaporation en deux étages présentant une pression absolue qui diminue du premier étage vers le deuxième étage de 100 mbars à 300 mbars jusqu'à 50 mbars à 150 mbars,
ii) la chaleur qui est utilisée pour l'évaporation selon l'étape i) provient pour une partie d'au moins 90% de l'enthalpie de réaction de la nitruration et
iii) le mélange de produits obtenu dans la nitruration présente une température d'au moins 110°C.

2. Procédé selon la revendication 1, **caractérisé en ce que** le mélange de produits obtenu dans la nitruration présente une température d'au moins 120°C.

3. Procédé selon la revendication 1 ou 2, **caractérisé en ce que** la chaleur pour l'évaporation selon l'étape ii) provient pour une partie d'au moins 90% de l'enthalpie de réaction de la réaction de nitruration adiabatique.

4. Procédé selon la revendication 3, **caractérisé en ce que** la chaleur pour l'évaporation selon l'étape ii) provient uniquement de l'enthalpie de réaction de la réaction de nitruration adiabatique.

5. Procédé selon la revendication 3, **caractérisé en ce qu'**une proportion de jusqu'à 10% de la chaleur utilisée pour l'évaporation dans les deux étages est introduite dans le procédé à partir de l'extérieur.

6. Procédé selon l'une quelconque des revendications précédentes, **caractérisé en ce que** l'alimentation du premier étage selon i) en acide résiduaire contient 60 à 75% en masse de H₂SO₄.

7. Procédé selon l'une quelconque des revendications précédentes, **caractérisé en ce que** la vapeur séparée dans chacun des étages selon i) est liquéfiée par condensation.

8. Procédé selon la revendication 7, **caractérisé en ce que** la vapeur séparée, liquéfiée est introduite dans un dispositif de séparation de phases.

9. Procédé selon l'une quelconque des revendications précédentes, **caractérisé en ce que** l'acide sulfurique concentré selon l'étape c), après la réalisation des deux étages de l'étape i), présente une proportion massique de H₂SO₄ qui n'est pas inférieure à 65%.

10. Installation pour la préparation de nitrobenzène, constituée par un réacteur (1), un dispositif de séparation de phases (2), deux dispositifs d'évaporation et de condensation reliés à des installations de vide (7, 9) et le cas échéant un récipient de stockage (5),
**caractérisée en ce que**
l'installation présente un premier évaporateur (3), pourvu d'un premier condensateur (8) associé et d'une installation de vide (9) raccordée, et un deuxième évaporateur (4), pourvu d'un deuxième condensateur (6) associé et d'une installation de vide (7) raccordée, et le premier évaporateur (3) étant raccordé directement au deuxième évaporateur (4) pour le passage de proportions non évaporées et la surface d'échange thermique du premier condensateur (8) étant en outre inférieure ou égale à deux fois la surface d'échange thermique du deuxième condensateur (6).
